# EUROPEAN PATENT APPLICATION

(11) **EP 4 389 165 A1**
(43) Date of publication of application: **26.06.2024**
(21) Application number: 22214892.6
(22) Date of filing: 20.12.2022
(51) Int. Cl.: A61M 1/06

(54) **PRESSURE CONTROL ARRANGEMENT FOR A MILK COLLECTION SYSTEM**

(71) Applicant: Koninklijke Philips N.V., 5656 AG Eindhoven (NL)
(72) Inventor: VAN BENTUM, Jesper William, Eindhoven (NL); LIPSCH, Job, Eindhoven (NL); VAN DER KOOI, Johannes Tseard, Eindhoven (NL); PALERO, Jonathan Alambra, Eindhoven (NL); VAN DEN BIJGAART, Adrianus Wilhelmus Dionisius Maria, Eindhoven (NL)
(74) Representative: Philips Intellectual Property & Standards

(57) **Abstract**

A pressure control arrangement for a milk collection system comprises a body for placing over a nipple of a user thereby to form at least a milk extraction chamber over the nipple. At least a part of the body is elastically deformable such that a volume of, or a volume in fluid communication with, the milk extraction chamber can be reduced by manually deforming the body thereby to create an under-pressure. The body is configured to retain a deformed state when an under-pressure within the milk extraction chamber exceeds a threshold under-pressure. A transition between a rest state and the deformed state of the body comprises a predetermined visually or audibly identifiable change in shape of the body.

## Description

### FIELD OF THE INVENTION

This invention relates to milk collection systems, for collecting milk from a lactating mother.

### BACKGROUND OF THE INVENTION

The best source of nutrition for a baby is human milk given by a breastfeeding mother. The world health organization recommends to breast feed for at least for the first six months of life, followed by continued breastfeeding with appropriate complementary foods for up to 2 years and beyond.

However, typically mothers often go back to work after already several weeks or months. To provide the best nutrition to their babies, mothers express milk using a breast pump. The expressed milk can be stored and given to the baby at a later stage and/or by somebody else.

Breast pumps typically unit make use of a drive arrangement for controlling an expression function. The drive arrangement has a pump for generating a vacuum in a vacuum chamber of the breast pump. A flexible membrane known as the diaphragm is located in the vacuum chamber. The pump is controlled to apply a vacuum to the diaphragm so that it deforms to create a vacuum in a breast receiving funnel, when it is sealed to the breast. This in turn applies a vacuum to the breast which enables milk to be expressed.

The vacuum is applied to the breast at intervals. That is, a pressure differential is applied on a cyclic basis. There are also wearable breast pumps, which are for example for mounting within a feeding bra.

Recently, there is a trend towards wearable passive milk collectors that collect leaking milk. The wearable passive milk collector can only be used with the baby present and feeding on the other breast.

When the baby starts to suckle at a breast, nipple stimulation causes the milk release (MER) at both breasts. The milk collector then collects milk from the other breast through a passive constant negative pressure (vacuum). The milk collector is for example formed as a compressible milk container which can be pressed inwards for generating a base vacuum for enhancing the milk extraction. It can also provide a holding pressure against the breast, although this may be achieved by separate means.

When the user compresses the milk collector and places the collector on her breast to generate under-pressure, it is important that the right amount of under-pressure is generated to reach a MER and more importantly, to extract the milk from her breast after the MER.

However, currently available milk collectors do not provide any feedback about the generation of a sufficient starting under-pressure level, which could lead to users not making optimal use of the milk collector. As a possible result, the MER may not be reached or the time to MER takes longer, and no milk or too little milk is collected.

It would be desirable to provide the user with feedback on achieving a suitable under-pressure.

### SUMMARY OF THE INVENTION

The invention is defined by the claims.

According to examples in accordance with an aspect of the invention, there is provided a pressure control arrangement for a milk extraction system comprising:
a body having a portion for placing over a nipple of a user thereby to form a milk extraction chamber, wherein at least a part of the body is elastically deformable such that a volume of, or a volume in fluid communication with, the milk extraction chamber can be reduced by manually deforming the at least part of the body thereby to create an under-pressure within the milk extraction chamber,
wherein the elastically deformable part of the body comprises a deformation feature at which the body preferentially deforms when the body transitions between a rest state and a deformed state, wherein the body is configured to retain the deformed state when an under-pressure within the milk extraction chamber exceeds a threshold under-pressure, and wherein transitions between the rest state and the deformed state comprise a predetermined visually and/or audibly and/or haptically identifiable change in shape of the body.

This pressure control arrangement is for use with a milk collection system for passive collection of leaking milk from the breast of a breastfeeding mother. The milk collection system is passive in the sense that there is no generation of a complex cyclic waveform. An under-pressure level is used, but it is generated manually, so there is no need for an electric or mechanical pump, and hence no need for a controller, or any actuators.

The manual deformation of the body is used to create an under-pressure, by creating a reduced volume of, or coupled to, the milk extraction chamber over the nipple (and optionally over the breast). Air for example leaks out from the interface between the body and the breast during this compression of the body, but the seal against the nipple or breast prevents air returning. An elasticity of the body means it tries to return to its original shape, and this returning force creates the under-pressure.

The reduced volume may be the volume over the nipple itself (i.e. the milk extraction chamber) or it may be a separate volume which is fluidly coupled to the milk extraction chamber so that there is at least partial equilibration of the pressure between that separate volume and the volume of the milk extraction chamber. Thus, controlling the pressure in the separate volume provides control of the pressure in the milk extraction chamber. For example, there may be a pressure control chamber (which includes the elastically deformable part) and a milk extraction chamber, coupled by a tube.

A pressure within a range of under-pressure levels is desired, sufficient for promoting milk extraction but not so great as to cause bruising. In accordance with the invention, a reduction in volume of a chamber formed by the body which is sufficient to create a desired under-pressure level in the milk extraction chamber, is achieved by a visually or audibly identifiable change in the shape of the body. Furthermore, while the under-pressure level remains within the desired range (in particular a sufficiently deep vacuum), the changed shape is retained.

The elastically deformable part of the body is for example configured to buckle at the deformation feature. This provides a bistable type deformation response, rather than simply a gradual change in shape.

For example, there may be collapsed of parts of the body, or inversion of portions of the body from convex to concave. These changes may be visible in use. Instead, there may be an audible click when the body is deformed from a rest state to a deformed state, and the under-pressure will maintain the body in the deformed state while the under-pressure remains at the desired level (i.e. within the desired range). Similarly, there may be a tactile signal generated when the body is deformed from the rest state to the deformed state.

In all cases, a similar visual, audible or tactile signal may be generated when the body returns from the deformed state to the rest state, for example because the under-pressure has dropped (i.e. the pressure has increased towards atmospheric pressure) during use of the milk collection system. In particular, when the milk is extracted, the presence of milk in the milk extraction chamber will result in a reduction in the level of under-pressure (i.e. an increase in pressure). When the pressure becomes outside the desired level (i.e. the under-pressure level is below a threshold), the shape will revert back from deformed second state to the rest state. This will also create the visually or audibly identifiable change in shape.

Thus, the change is shape is used both for the user to know that the initially applied deformation will be sufficient to induce the desired under-pressure level (i.e. a transition from the rest state to the deformed state), and also to alert the user that the under-pressure level has dropped below a threshold (i.e. a transition from the deformed state back to the rest state).

A change in visual appearance is typically a change in shape and/or a change in surface decoration/appearance. When the visually changed appearance is present (i.e. the deformed state), the user can know that the required under-pressure level is present for correction functioning of the system.

An audible change is for example a sound made when the shape toggles between two distinct shapes, for example a buckling or un-buckling change in shape. Thus, the sound indicates when the shape has been deformed into the shape suitable for the desired under-pressure level and the sound also indicates when the shape has deformed back because the under-pressure level has dropped below the threshold (i.e. the pressure has increased closer to ambient pressure).

A haptic change is for example that a mechanical limit is reaches as to how far volume can be reduced, so that at that point the elastic part of the body is much more resistant (so the user applies a manual force until the force is no longer sufficient to result in a deformation and hence volume change). This haptic change may thus be felt when applying a manual force, but equally it may be felt when the under-pressure drops as a result of milk in the milk extraction chamber.

It is noted that the body (or a part of the body) may be elastically deformable by virtue of the material of the body or that part of the body (e.g. rubber) but there may be separate elements to provide the desired elasticity, such as springs.

The threshold under-pressure is for example in the range -4kPa to -18kPa, such as -6kPa to -12kPa. These ranges are suitable for milk extraction without causing pain.

The deformation feature for example comprises a set of one or more ribs. The ribs provide zones where the body will preferentially deform in response to an internal under-pressure in the milk extraction chamber.

The deformation feature for example comprises a rib which forms a closed shape, wherein the area of the body within the closed shape is configured to be inverted when the threshold under-pressure is present. Thus, a portion of the shape of the body may transition from a outward, convex, shape, to an inward, concave, shape. This provides a striking change in visual appearance and/or provides a transition sound such as a clicking sound.

The deformation feature for example instead comprises a collapse zone which forms a closed shape, wherein the collapse zone is configured to be collapsed when the threshold under-pressure is present. In this way, a portion of the body is present when there is insufficient under-pressure but it is no longer visible (or less visible) when the desired under-pressure is present.

The deformation feature for example comprises a set of the collapse zones which form a set of concentric closed shapes, wherein the collapse zones are configured to be collapsed when the threshold under-pressure is present.

In this case, different sets of the collapse zones may be configured to be collapsed when different levels of under-pressure are present. In this way, the configuration may enable information about multiple pressure levels to notified to the user.

The collapse zone or collapse zones for example have a contrasting color with the remainder of the body. Thus, when not collapsed, there may be bright color band, whereas when collapsed, those color bands disappear or substantially disappear.

In all examples above, elastically deformable part of the body is for example configured to deform to the deformed state when a predetermined manual force is applied to the elastically deformable part of the body. In other words, the user applies a manual force to deform the body into the pressure- reducing deformed state, and the resulting under-pressure created if it reaches the threshold under-pressure level, means the body retains that deformed shape.

The pressure control arrangement may comprise an outer frame over the body. This can prevent snagging of clothes or a bra on the body.

A pressure sensor may be provided within the body, and a display unit for displaying an indication of the sensed pressure. This enables a pressure measurement rather than only an indication of whether a threshold under-pressure is reached.

The display unit for example comprises a light emitting or sound emitting or vibration generating arrangement. An example is an LED arrangement.

The invention also provides a milk collection system comprising the pressure control arrangement defined above. The milk extraction chamber either forms the milk collection chamber, or it is connected to a separate milk collection chamber.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a first example of a milk collection system;
Fig. 2 shows a second example of a milk collection system; and
Fig. 3 shows a third example of a milk collection system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a pressure control arrangement for a milk collection system, which comprises a body for placing over a nipple of a user thereby to form at least a milk extraction chamber over the nipple. At least a part of the body is elastically deformable such that a volume of, or a volume in fluid communication with, the milk extraction chamber can be reduced by manually deforming the body thereby to create an under-pressure. The body is configured to retain a deformed state when an under-pressure within the milk extraction chamber exceeds a threshold under-pressure. A transition between a rest state and the deformed state of the body comprises a predetermined visually or audibly identifiable change in shape of the body.

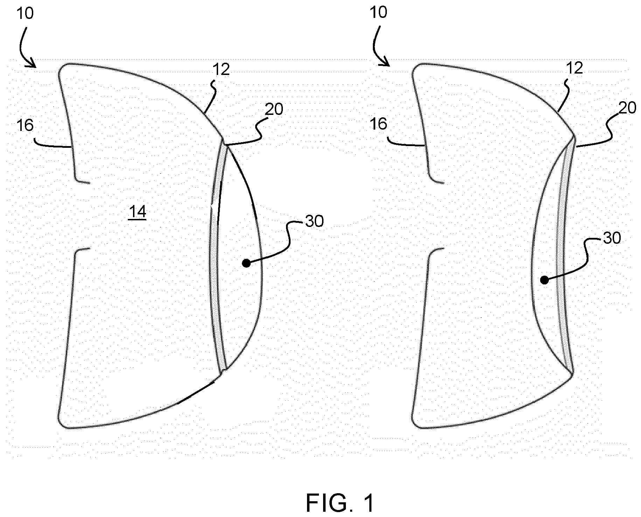

Fig. 1 shows a first example of a milk collection system which incorporates a pressure control arrangement. The left image shows the system in a rest state and the right image shows the system in a deformed state after manual application of force to a body of the pressure control arrangement to create an under-pressure.

The milk collection system 10 comprises a body 12, which in this example defines a single housing for placing over a nipple of a user thereby to form a milk extraction chamber 14 over the breast.

The primary purpose of the milk extraction chamber is to apply an under-pressure (i.e. a pressure below atmospheric pressure) to the nipple of the user. This under-pressure promotes milk extraction, and is for example used once the milk ejection reflex has been stimulated on the other breast.

In the example shown, the milk extraction chamber is a milk collection chamber, i.e. the milk is extracted directly into a milk collection vessel. However, the milk collection vessel and the milk extraction chamber may be separate devices, with a fluid connection between the milk extraction chamber and the milk collection chamber. The separate milk extraction and milk collection chambers may be at the same pressure, or else there may be a valve between them so that only the milk extraction chamber is operated at a reduced pressure.

Furthermore, the body 12 may instead have different parts. For example one portion may form the milk extraction chamber and be placed over the nipple, whereas another portion of the body may be remote from the nipple area, and it may be the part which is actuated to create the desired under-pressure. For example, there may be a first chamber formed over the nipple which is the milk extraction chamber and a second chamber formed remotely, and which functions as the pressure control chamber. The two chambers are then coupled together so that they are at the same, or substantially the same, pressure. The pressure control chamber is then elastically deformable, or has at a least a part which is elastically deformable to adjust the volume of the pressure control chamber (and hence the combined volume of the pressure control chamber and the milk extraction chamber),

In this example (wherein the housing forms a single chamber which is the milk collection chamber) the housing has a lip 16 which is deformed over the breast to create a seal with the breast tissue when the body is positioned over the breast.

The body, or at least a part of it, is elastically deformable, so that it has a rest state as shown in the left image. By manually deforming the body (or the part of it which is designed for that purpose), e.g. by squeezing or pushing against a portion of the body, the volume of the milk extraction chamber 14 is reduced. Air is pushed past the lip 16 but cannot flow the other way. Thus, the air volume is reduced, and when the body tries to return to its original shape due to its elasticity, an under-pressure is created within the milk extraction chamber defined by the body.

The milk collection system is passive, with no active (e.g. electrical) generation of a complex cyclic waveform.

To the extent described above, the functionality is known.

The invention provides a deformation feature 20 at which the body (i.e. the elastic part of the body) preferentially deforms when there is an under-pressure within the chamber or chambers defined by the body.

In particular, the under-pressure should be maintained within a desired range. The maximum under-pressure level may be limited by the designed maximum deformation of the body (i.e. there is a maximally deformed state) corresponding to a maximum amount of volume reduction. Alternatively, a valve may be used to set a maximum level of under-pressure (i.e. a minimum pressure).

The body is designed to convey to the user an indication of when the minimum under-pressure is reached or is present. This the point at which the under-pressure level becomes insufficient, and the user should re-pressurize the system.

The right image shows a deformed state of the body, and one in which a desired level of under-pressure is present (in particular, the level of under-pressure exceeds a minimum threshold). When transitioning from a body shape/volume which corresponds to insufficient under-pressure to a body shape/volume which corresponds to sufficient under-pressure, there is a visually and/or audibly identifiable change in shape of the body. Thus, the user knows when applying the milk collection system that they have performed the manual deformation in a way which is sufficient to provide the desired level of under-pressure.

For the example of Fig. 1, the deformation feature comprises a rib. The rib 20 is made of a compressible and deformable material. The rib provides a zone where the body will preferentially deform, and that deformation will be held by a sufficient internal under-pressure.

The rib 20 forms a closed shape, shown as an oval near the front of the body. The area 30 of the body within the closed shape can be inverted by a user manually pushing on the area 30. This provides a striking change in visual appearance and may also provide an audible click. It results in a buckling type shape change, which is essentially bistable in nature. This creates an under-pressure, and while a sufficient under-pressure is present, the deformed shape is retained. Thus, the area 30 inverts from a outward, convex, shape, to an inward, concave, shape and retains the inward shape while sufficient under-pressure is present.

The body may be deformed by inducing the same change in shape, hence in this example pushing inwardly on the area 30 as explained above. However, in other examples, the body may be deformed by applying other forces, e.g. squeezing the sides. Once the external manual force is removed, the natural state of the body (retaining the particular under-pressure present) may then become the shape shown to the right of Fig. 1, which again indicates to the user that sufficient deformation has been performed.

When the level of under-pressure drops, for example because milk is filling the space in the milk extraction chamber, the shape will revert back to the original shape once the level of under-pressure passes a threshold. This will again cause the same visually and/or audibly identifiable change in shape. Thus, the user is notified that they need to re-pressurize the system.

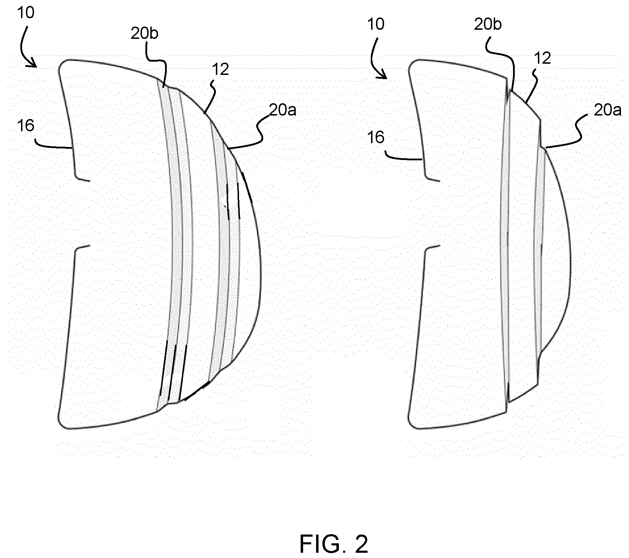

Fig. 2 shows a second example of a milk collection system. The left image again shows the system in a rest state and the right image shows the system after manual application of force to the body to create an under-pressure.

For the example of Fig. 2, the deformation feature 20 comprises collapse zones, such as a bellows. There may be a single such zone although two are shown. There may be more than two. The collapses zone or zones again form a closed shape, shown as an concentric ovals near the front of the body. The collapse zones remain collapsed in response to under-pressure levels. In this way, a portion of the body is present when there is no under-pressure but it is no longer visible (or less visible) when the under-pressure is present.

The collapse zones may all remain collapsed when a first (maximum) threshold under-pressure is present. However, different sets of the collapse zones may also be configured to retain their collapsed states in response to different levels of under-pressure.

For example, a first collapse zone may retain its collapsed state when a first under-pressure level is present, whereas both collapse zones may retain their collapsed states when a second (deeper vacuum) under-pressure level is reached. In this way, the configuration may enable information about multiple pressure levels to notified to the user.

The collapse zone or collapse zones for example have a contrasting color with the remainder of the body, e.g. a bright color for the collapse zones and a dull color for the rest of the body. Thus, when not collapsed, there may be a bright color band or bands, whereas when collapsed, those color bands disappear or substantially disappear.

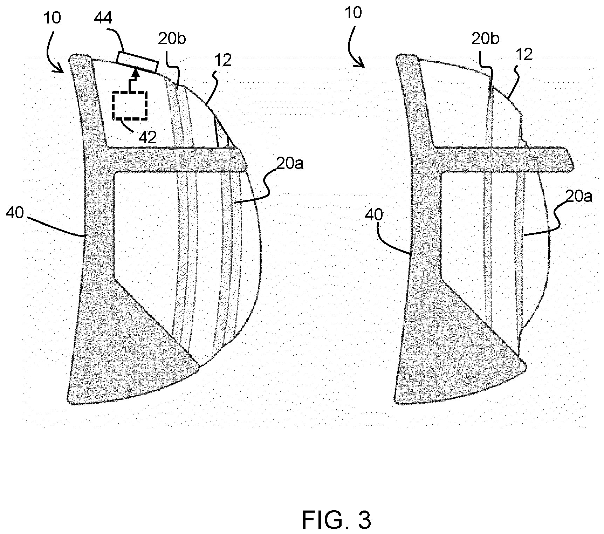

Fig. 3 shows a third example of a milk collection system. The left image again shows the system in a rest state and the right image shows the system after manual application of force to the body to create an under-pressure.

This is shown as a modification to the design of Fig. 2 but it could equally be applied to Fig. 1. An outer frame 40 is provided over the body. The frame provides a hard shell, which prevents the user's bra or clothing from interfering with the milk collector's pressure profile.

A pressure sensor 42 may be provided within the body, and a display unit 44 is provided on the outside for displaying an indication of the sensed pressure. This enables a pressure measurement rather than only an indication of whether a threshold under-pressure is reached.

The display unit 44 for example comprises an LED arrangement, for example with different color LEDs for different pressure levels (e.g. red, amber and green to indicate the suitability of the under-pressure level).

The examples above shows that the controllable deformation of the body could be a nonlinear or linear elastic deformation.

The examples above are based on a deformable body which defines the external appearance of the milk collection system. However, in other examples, the deformable body may be covered by other parts which are then actuated by the user to induce the deformation of the body. For example, a rotary knob may be provided which operates a plunger (e.g. to push on the area 30 in Fig. 1). The body itself may be twisted, e.g. on a threaded shaft, to induce its change in volume.

Thus, the user does not necessarily interact directly with the body, but the manual deformation may instead be indirect, in that the manual interaction of the user is with another part of the pressure control arrangement. There is still however manual deformation of the body in that no independently powered actuator is used.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A pressure control arrangement for a milk extraction system (10) comprising:
a body (12) having a portion for placing over a nipple of a user thereby to form a milk extraction chamber (14), wherein at least a part of the body (12) is elastically deformable such that a volume of, or a volume in fluid communication with, the milk extraction chamber (14) can be reduced by manually deforming the at least part of the body thereby to create an under-pressure within the milk extraction chamber (14),
wherein the elastically deformable part of the body (12) comprises a deformation feature (20) at which the body preferentially deforms when the body transitions between a rest state and a deformed state, wherein the body is configured to retain the deformed state when an under-pressure within the milk extraction chamber (14) exceeds a threshold under-pressure, and wherein transitions between the rest state and the deformed state comprise a predetermined visually and/or audibly and/or haptically identifiable change in shape of the body.

2. The pressure control arrangement of claim 1, wherein the threshold under-pressure is in the range -4kPa to -18kPa, such as -6kPa to -12kPa.

3. The pressure control arrangement of any one of claims 1 to 2, wherein the elastically deformable part of the body is configured to buckle at the deformation feature.

4. The pressure control arrangement of any one of claims 1 to 3, wherein the deformation feature (20) comprises a set of one or more ribs.

5. The pressure control arrangement of claim 4, wherein the deformation feature (20) comprises a rib which forms a closed shape, wherein the area (30) of the body within the closed shape is configured to retain the deformed state, in the form of an inverted shape, at the threshold under-pressure.

6. The pressure control arrangement of claim 4, wherein the deformation feature (20) comprises a collapse zone (20a, 20b) which forms a closed shape, wherein the collapse zone is configured to retain the deformed state, in the form of a collapsed shape, at the threshold under-pressure.

7. The pressure control arrangement of claim 6, wherein the deformation feature comprises a set of collapse zones (20a, 20b) which form a set of concentric closed shapes, wherein the collapse zones are configured to retain the deformed state, in the form of collapsed shapes, at the threshold under-pressure.

8. The pressure control arrangement of claim 7, wherein different sets of the collapse zones (20a, 20b) are configured to retain collapsed shapes at different levels of under-pressure.

9. The pressure control arrangement of any one of claims 6 to 8, wherein the collapse zone or collapse zones (20a, 20b) have a contrasting color with the remainder of the body.

10. The pressure control arrangement of any one of claims 1 to 9, wherein the elastically deformable part of the body (12) is configured to deform to the deformed state of the body when a predetermined manual force is applied to the elastically deformable part of the body.

11. The pressure control arrangement of any one of claims 1 to 10, comprising an outer frame (40) over the body.

12. The pressure control arrangement of any one of claims 1 to 11, further comprising a pressure sensor (42) within a chamber formed by the body, and a display unit (44) for displaying an indication of the sensed pressure.

13. The pressure control arrangement of claim 12, wherein the display unit (44) comprises a light emitting or sound emitting or vibration generating arrangement.

14. A milk collection system comprising the pressure control arrangement of any one of claims 1 to 13.

15. The milk collection system of claim 14, wherein:
the milk extraction chamber forms the milk collection chamber; or
the milk extraction chamber is connected to a separate milk collection chamber.
